# EUROPEAN PATENT SPECIFICATION

(11) **EP 0 970 669 B1**
(45) Date of publication and mention of the grant of the patent: **21.05.2003**
(21) Application number: 99111876.1
(22) Date of filing: 21.06.1999
(51) Int. Cl.: A61F 5/01

(54) **Elbow brace with movable strap**
Ellbogenstütze mit bewegbarer Lasche
Support pour le coude avec bande mobile

(30) Priority: 10.07.1998 US 92389 P
(43) Date of publication of application: 12.01.2000
(73) Proprietor: Becton, Dickinson and Company, Franklin Lakes, New Jersey 07417 (US)
(72) Inventor: Madow, Stephen, Swampscott, MA 01907 (US); Kausek, Jim, Swampscott, MA 01907 (US)
(74) Representative: von Kreisler, Alek, Dipl.-Chem.

(56) References cited:
- WO-A-95/33426
- WO-A-97/12570

## Description

### BACKGROUND OF THE INVENTION

### 1. Field of the Invention

The present invention relates to an elbow brace and, more particularly, to a laminated elbow brace made from a unique blend of material that provides heat retention, compression with breathability, and wicking of perspiration away from the skin to dry the skin area quickly having a movable support.

### 2. Background Description

A wide variety of externally applied elastic supports and rigid, joint-immobilizing braces are known to protect healthy and injured joints and to promote healing of certain injuries. Supports and braces are commonly used for injuries and other medical problems at the knee, thighs, elbow, waist, wrist and back. Common injuries that can be helped by a support include strained or torn ligaments, tendentious, arthritis, and pulled or strained muscles.

Plastic or "soft" supports are usually preferred over braces where the body part is generally healthy and the intent is to support it in order to prevent injury of a joint and surrounding tissue. Soft supports are also used to protect and promote the healing of injured members where there are no broken bones and the patient is mobile. A support may be worn, for example, before engaging in work or a sports activity that is expected to involve unusual stretching or load bearing. The elasticity of the support is important not only to provide supporting externally applied compression, but also to maintain the support in a selected position on the body. Ideally the support is constructed so that it flexes easily and interferes as little as possible with the normal range of motion of the body part. The elasticity of the support also accommodates changes in the size of the body part produced by physical exertion, changes in the condition of an injury (e.g., a reduction in swelling), or mere changes in the elevation of the body part, e.g., when an injured ankle is elevated.

The most common form of elastic support is a simple tubular sleeve of a stretch fabric such as the stretch nylon material used in ACE brand bandages and supports. Such a sleeve is pulled over and grips the body part to be protected as well as adjoining regions. When used on joints, the major problems are chafing and biting of the fabric during flexing, particularly at the interior of a joint such as the back of a knee or the "inside" of an elbow. Flexures of body parts and changes in body size can also result in a migration of the position of the support on the body.

An elbow brace, as described in the preamble of claim 1, is disclosed in WO9533426 A.

Currently available supports do not provide the breathability necessary to prevent perspiration from forming where the support and skin are in contact and also have designs such that there is a seam located against the skin that causes pinching or biting on the skin.

### SUMMARY OF THE INVENTION

The present invention overcomes the problems identified in the background material by providing a laminated elbow brace made from a unique blend of material that provides heat retention, compression with breathability, and quick wicking of perspiration away from the skin having a movable support.

In particular, the present invention provides a laminated elbow brace made from a unique blend of material combining Airpreene™ material with Coolmax™ material as a liner. Airpreene™ material affords the properties of heat retention, compression and breathability, while Coolmax™ material wicks perspiration away from the skin and dries the area quickly. The combination of these materials provides compression and heat retention to the interior portion of the elbow and comfort to the elbow.

These and other aspects, features and advantages of the present invention will become apparent from the following detailed description taken in conjunction with the accompanying drawings.

### DESCRIPTION OF THE DRAWINGS

Fig. 1 is a perspective view of an elbow brace according to the present invention with its movable support removed during use on a patient's arm;
Fig. 2 is another perspective view of the elbow brace shown in Fig. 1, with its movable support attached during use on a patient's arm;
Fig.3 is a cross-sectional view of a portion of the elbow brace and support shown in Fig. 2;

### DETAILED DESCRIPTION

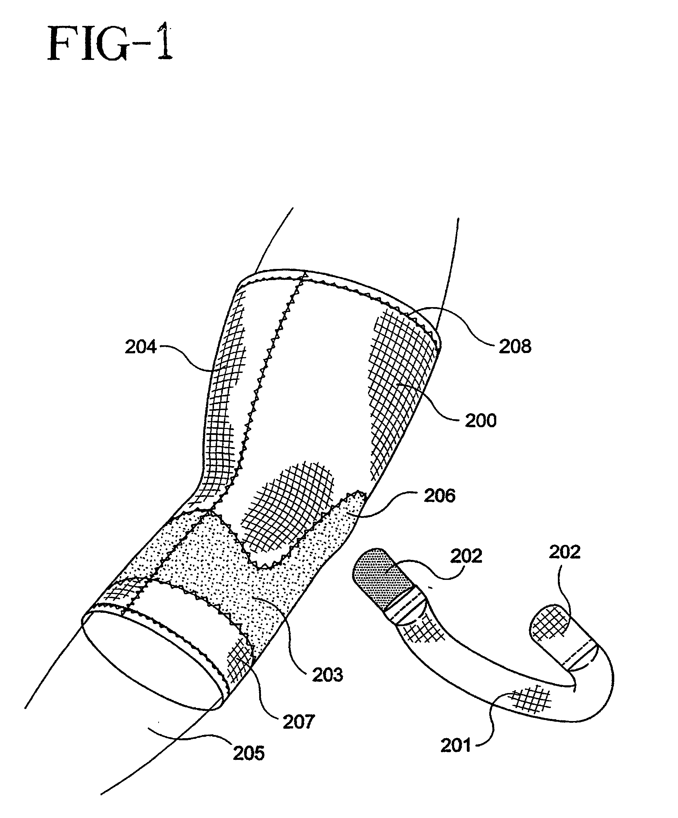

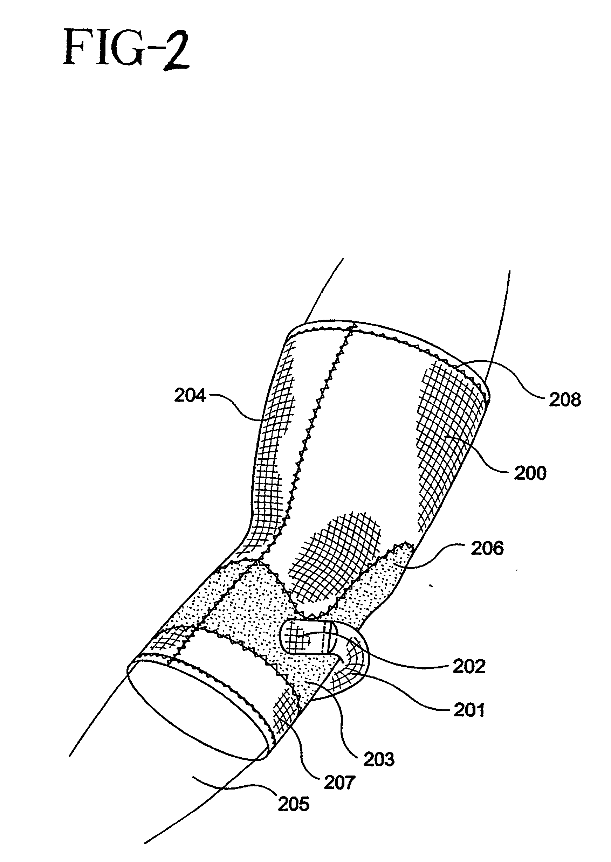

Fig. 1 is a perspective view of an elbow brace 200 according to the present invention with a movable support 201 removed during use on a patient's arm 205. As shown in Fig. 1, elbow brace 200 includes an upper section 204 and a lower section 207 with a special mesh section 203 therebetween encircling arm 205 and having an extension 206 extending up arm 205 adjacent the patient's elbow. Fig.2 is another perspective view of elbow brace 200 shown in Fig.1, with movable support 201 attached to mesh portion 203 during use on the patient's arm 205. Movable support 201 includes a pair of hook fasteners 202 at each end that are used to attach movable support 201 to the mesh portion of elbow brace 200. Movable support 201 also provides individual tendentious relief and is formed by a foam cylinder 211 that is stitched between an outer layer of material 201.

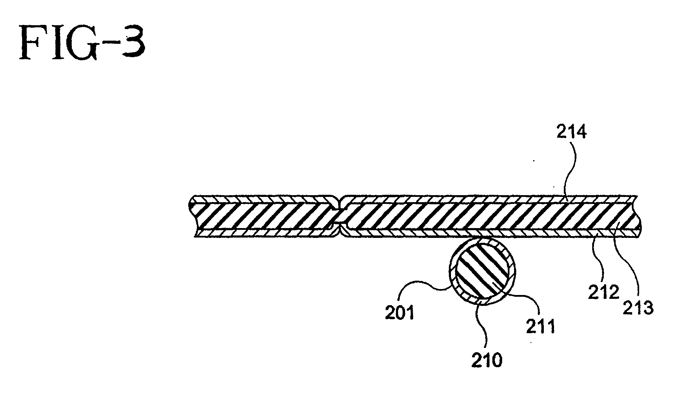

Fig.3 is a cross-sectional view of a portion of elbow brace 200 and movable support 201 shown in Fig.2. As shown in Fig.3 elbow brace 200 incorporates the same unique material concept of the present invention which combines Airpreene™ material with Coolmax™ material as a liner. The Airpreene™ material 213 having a mesh material 212 laminated on one side and the Coolmax™ material 214 laminated to the other side provides the property of heat retention and compression with breathability, while the Coolmax™ material 214 inside elbow brace 200 whisks perspiration away from the skin in the vicinity of the patient's elbow and dries the area quickly. The present invention also minimizes bulk in the elbow crease when bending and movable support 201 provides a unique design that is fully adjustable to provide specific pressure where needed while preventing circulation from being cut off since it does not circumvent the forearm. In addition, an elliptical seam 208 near the top of elbow brace 200 provides optimal comfort during arm contraction. The exemplary elbow brace 200 shown here uses an Airpreene™ material that is 1.5 mm thick where it is laminated to the Coolmax™ material and a 3 mm thickness in the upper and lower sections 204 and 207, respectively.

In the foregoing discussion, it is to be understood that the above-described embodiments of the present invention is simply illustrative of various features that can be used in a variety of orthopedic braces. Other suitable variations, modifications, applications, and combinations of these features could be made to or used in this embodiment and still remain within the scope of the present invention.

## Claims

1. An elbow brace (200) comprising:
an upper (204) and lower (207) section made from a lamination of material that provides heat retention and compression with breathability while also providing whisking of perspiration away from the patient's skin to dry the patient's skin area quickly;
and a center section (203) between said upper and lower sections for surrounding an elbow;
**characterised by**
a support (201) movably attachable to said center section to provide support to the elbow.

2. The elbow brace according to Claim 1, further comprising an inner shell made from said lamination of material.

3. The elbow brace according to Claim 2, wherein an inner layer of said inner shell is in contact with the patient's skin and is made of a material that whisks perspiration away from the skin to dry the skin quickly.

4. The elbow brace according to Claim 1, wherein said center section (203) includes a mesh fastener material for receiving a hook fastener material (202) on said movable support (201) to fasten said movable support to said center section and provide support to the elbow.

5. The elbow brace according to Claim 1, wherein said upper section (204) includes an elliptical seam (208) to provide optimal comfort during arm contraction.

## Patentansprüche

1. Ellbogenstütze (200) mit:
einem oberen (204) und einem unteren (207) Abschnitt aus einem Material-Laminat, das Wärmespeicherung und Kompression mit Atmungsfähigkeit ermöglicht, während es ferner ein Ableiten von Perspiration von der Haut des Patienten weg ermöglicht, um den Hautbereich des Patienten schnell zu trocknen;
und einem zwischen dem oberen und dem unteren Abschnitt angeordneten Mittelabschnitt (203) zum Umfassen eines Ellbogens;
**gekennzeichnet durch**
einem bewegbar an dem Mittelabschnitt angeordneten Support (201), um dem Ellbogen Halt zu geben.

2. Ellbogenstütze nach Anspruch 1, ferner mit einer inneren Schale, die aus dem Material-Laminat besteht.

3. Ellbogenstütze nach Anspruch 2, bei der sich eine innere Schicht der inneren Schale in Kontakt mit der Haut des Patienten befindet und aus einem Material besteht, das Perspiration von der Haut weg ableitet, um die Haut schnell zu trocknen.

4. Ellbogenstütze nach Anspruch 1, bei der der Mittelabschnitt (203) ein Netz-Befestigungsmaterial zur Aufnahme eines Klettverschluss-Befestigungsmaterials (202) an dem bewegbaren Support (201) aufweist, um den bewegbaren Support an dem Mittelabschnitt zu befestigen und dem Ellbogen Halt zu geben.

5. Ellbogenstütze nach Anspruch 1, bei der der obere Abschnitt (204) einen elliptischen Saum (208) aufweist, um während einer Arm-Kontraktion optimale Bequemlichkeit schaffen.

## Revendications

1. Coudière (200), comportant :
des tronçons supérieur (204) et inférieur (207) fabriqués à partir d'un stratifié de matériaux fournissant une retenue thermique et une compression avec une perméabilité à la transpiration, tout en fournissant également une évacuation de la transpiration loin de la peau du patient pour sécher une zone de peau du patient rapidement,
et un tronçon central (203) situé entre lesdits tronçons inférieur et supérieur pour entourer un coude,
**caractérisée par**
un support (201) pouvant être fixé de manière mobile sur ledit tronçon central pour fournir un support du coude.

2. Coudière selon la revendication 1, comportant de plus une enveloppe intérieure constituée dudit stratifié de matériau.

3. Coudière selon la revendication 2, dans laquelle une couche intérieure de ladite enveloppe intérieure est en contact avec la peau d'un patient, et est constituée d'un matériau qui évacue la transpiration loin de la peau pour sécher la peau rapidement.

4. Coudière selon la revendication 1, dans laquelle ledit tronçon central (203) comporte un matériau de fixation à mailles pour recevoir un matériau de fixation à crochets (202) sur ledit support mobile (101), afin de fixer ledit support mobile sur ledit tronçon central, et de fournir un support du coude.

5. Coudière selon la revendication 1, dans laquelle ledit tronçon supérieur (204) comporte une couture elliptique (208) pour fournir un confort optimum pendant une contraction du bras.
